Europäisches Patentamt

(19) European Patent Office

Office européen des brevets

(11) Publication number : **0 327 005 B1**

(12) # EUROPEAN PATENT SPECIFICATION

(45) Date of publication of patent specification :
**15.07.92 Bulletin 92/29**

(51) Int. Cl.⁵ : **A61M 1/00, A61M 1/34**

(21) Application number : **89101593.5**

(22) Date of filing : **31.01.89**

(54) **Process for extracorporeal treatment of ascitic fluid.**

(30) Priority : **02.02.88 IT 1928688**

(43) Date of publication of application :
**09.08.89 Bulletin 89/32**

(45) Publication of the grant of the patent :
**15.07.92 Bulletin 92/29**

(84) Designated Contracting States :
**AT BE CH DE ES FR GB GR IT LI LU NL SE**

(56) References cited :
EP-A- 0 228 160
DE-A- 3 524 823
DE-B- 2 017 473
US-A- 4 083 786
US-A- 4 416 772
US-A- 4 708 713

(73) Proprietor : **DIDECO S.p.A.**
**Via Galilei 3**
**I-41037 Mirandola (Province of Modena) (IT)**

(72) Inventor : **Valbonesi, Mauro**
**Via P. B. Shelley 59/4**
**I-16100 Genova (IT)**

(74) Representative : **Modiano, Guido et al**
**MODIANO, JOSIF, PISANTY & STAUB**
**Modiano & Associati Via Meravigli, 16**
**I-20123 Milano (IT)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 327 005 B1

## Description

The present invention relates to a process for the extracorporeal treatment of ascitic fluid.

It is known that the optimum treatment of the disease known as refractory ascites consists of the exploitation of the ascitic fluid taken from a patient's abdominal cavity and suitably treated as will be described hereinafter; the reasons which lead to the preference of this form of treatment are several, both medical and economical.

Working in this direction, techniques have been developed for the treatment of the ascitic fluid taken from the patient which essentially consist of passing it through a concentrator filter, inside which said fluid loses water before being utilized.

This technique, however, has an obvious disadvantageous feature, residing in the fact that the treated fluid, besides the substances which must be utilized, which essentially consist of proteins and chiefly of albumin, also contains noxious substances such as, above all, blood coagulation factors, which are made particularly active by exposure to the filtering surface: this causes an extremely hazardous procoagulating activity in the utilized fluid.

But other noxious substances are present in the fluid treated according to the described prior art, such as for example fragments of cells which are seen by the immune system as foreign matter such as to cause even considerable feverish reactions.

US-A-4 083 786 discloses a treatment of ascitic fluid consisting of the removal of blood, cancer cells and bacteria by filtration and further consisting of the concentration of the fluid by filtration.

The aim of the present invention is to devise a process for the treatment of ascitic fluid which allows one to utilize exclusively the desired substances, excluding endotoxins, activated coagulation factors, aromatic amino acids, bilirubin.

The proposed aim is achieved by a process for the extracorporeal treatment of ascitic fluid, according to the invention, comprising a first step in which the fluid is passed through a filter which is capable of eliminating from the filtrate the particles, representing noxious substances, having a molecular weight greater than that of the substances which it is desired to exploit, and a second step in which the filtrate of the first step is concentrated in a suitable filter, with loss of water, characterized in that

the molecular weight discriminating ability, or cut-off, of the filter used in the first step has a value of approximately 200,000 daltons, while the cut-off of the filter used in the second step has a value of approximately 30,000 daltons.

Further characteristics and advantages of the invention will become apparent from the description of a preferred, but not exclusive, embodiment of the invention, given only by way of non-limitative example, which makes reference to the accompanying drawing in which the only figure shows a schematic flow diagram of the process of this invention.

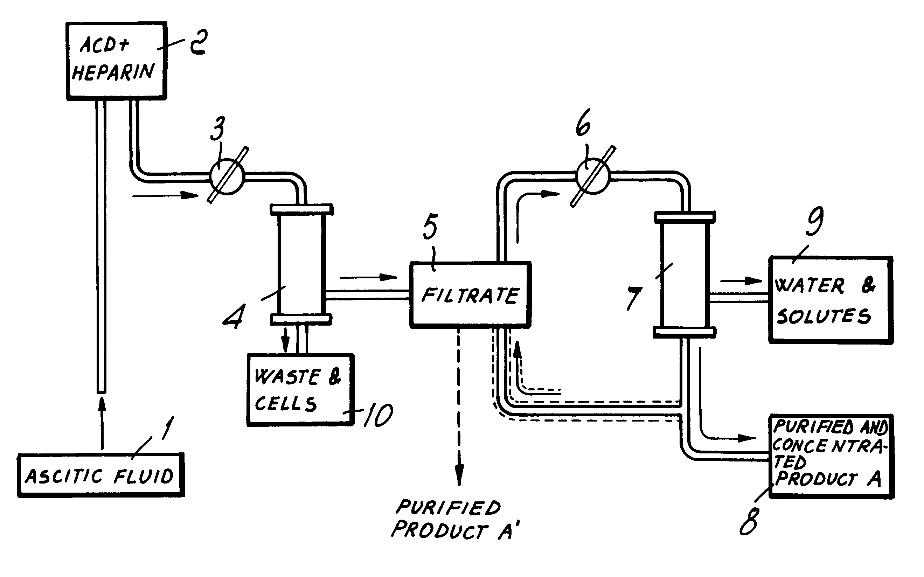

Referring to Fig. 1, the ascitic fluid 1 taken from a source of ascitic liquid, formed by an aqueous solution of proteinic substances, primarily including albumin, which are to be utilized, and of noxious substances, such as essentially endotoxins and activated coagulation factors, is added e.g. by gravity to a bag 2 in which anticoagulating substances, such as ACD and/or heparin, are added, and is drawn from said bag 2 by means of peristaltic pump 3 in order to be sent to a filter 4 in which the first step of the process is performed.

Said filter is an ordinary, per se known filter for example of the type with hollow fibers of cellulose acetate, which such a porosity as to provide a molecular weight discriminating ability, known as cut-off, equal to approximately 200,000 daltons: this means that this filter retains all particles having a molecular weight greater than approximately 200,000 daltons, corresponding to the molecular weight range for the noxious substances which it is desired to eliminate before utilizing the fluid. The retained particles are collected into a waste bag 10 for eventual disposed. Suitable for the purpose is a filter manufactured by the applicant and identified by the catalogue No. BT901 and commercialized under the tradename "ALBUSAVE"®.

The fluid exiting from the described filter and continuing through the process is thus composed of a very diluted aqueous solution of proteinic substances, and one may say, simplifying, of albumin.

Said fluid reaches a bag 5 from which it is drawn by means of a pump 6 to be sent to the filter 7 in which the second step of the process is performed, and which consists of an ordinary hemofilter, for example of the type with hollow fibers of polysulfone or cellulose acetate, having a cut-off equal to approximately 30,000 daltons and therefore such as to offer great permeability to water and to solutes having a molecular weight of up to approximately 30,000 daltons, which are collected into a waste bag 9 for eventual disposal, retaining albumin, which therefore exits from this filter in a concentrated aqueous solution, which is collected into a bag 8 as product A. Suitable for the purpose is the filter manufactured by the applicant and identified by the catalogue No. BT920 and under the tradename "EMOCONCENTRATORE DIDECO"®. Suitable for the purpose may be also the filter known under the tradename of "PAN 15"® of the firm Asai, Tokyo.

At the exit from the filter now described, the process provides the possibility of utilizing the albumin in concentrated aqueous solution directly as product, but it is also possible to recycle said solution from the filter 7 to the bag 5 arranged upstream with respect to said filter as indicated by dashed lines; in this case, the solution can be withdrawn from bag 5 as product A as indicated by dashed lines.

From what has been described, it is therefore apparent that the process according to the invention allows one to provide an extracorporeal treatment of the ascitic fluid at the end whereof the optimum condition is achieved of providing for use a concentrated aqueous solution of albumin, having optimum characteristics, and it is important to note the simplicity of the process itself: in fact, it is achieved by means of filters which are extremely common in the art, which are employed herein in a particular manner.

Another very important characteristic of the process according to the invention resides in the fact that the proteinic substances which are to be utilized are for the entire process in a state of aqueous solution such as to allow a correct treatment thereof.

The exploitation of the treated fluid, obtained with the treatment of the present invention, may be carried out in ways that are outside the scope of the present invention which is limited to the preparation of an ascitic fluid ready for utilization, not excluding the preservation of the treated fluid in air conditioned environments according to optimal sanitary conditions.

The invention described is susceptible to numerous modifications and variations, within the scope of the inventive concept, as defined by the following claims.

When technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Process for the extracorporeal treatment of ascitic fluid, comprising a first step in which the fluid is passed through a filter which is capable of eliminating from the filtrate particles, representing noxious substances, having a molecular weight greater than that of the substances which it is desired to exploit, and a second step in which the filtrate of the first step is concentrated in a suitable filter with loss of water, characterized in that the molecular weight discriminating ability, or cut-off, of the filter used in the first step has a value of approximately 200,000 daltons, while the cut-off of the filter used in the second step has a value of approximately 30,000 daltons.

2. Process according to claim 1, characterized by the presence of a bag which receives the ascitic fluid into which an anticoagulating substance is added, a pump being provided which is adapted for the transfer of the ascitic fluid from said bag to the filter used in the first step.

3. Process according to one or more of the preceding claims, characterized by the presence of a bag on the output line of the filter used in the first step, from which the ascitic fluid is drawn by a pump to be sent to the filter used in the second step.

4. Process according to one or more of the preceding claims, characterized in that the purified and concentrated ascitic fluid is utilized after passing through the filter employed in the second step.

5. Process according to one or more of the preceding claims, characterized in that the ascitic fluid is recirculated from the filter employed in the second step to the bag interposed between the filters employed respectively in the first and in the second step and then is withdrawn from said bag as product.

## Patentansprüche

1. Verfahren zur extrakorporalen Behandlung von Aszitesflüssigkeit, enthaltend einen ersten Verfahrensschritt, in dem die Flüssigkeit durch einen Filter gegeben wird, der die Abscheidung von Festkörpern aus dem Filtrat ermöglicht, die Schadstoffe darstellen und ein Molekulargewicht aufweisen, das größer ist als das der Substanzen, die ausgenutzt werden sollen, und enthaltend einen zweiten Verfahrensschritt, in dem das Filtrat des ersten Verfahrensschritts in einem geeigneten Filter entwässert wird, dadurch gekennzeichnet, daß das Molekulargewichts-Diskriminierungsvermögen oder Cut-Off des im ersten Verfahrensschritt verwendeten Filters einen Wert von etwa 200,000 Dalton aufweist, während der Cut-Off des im zweiten Verfahrensschritt verwendeten Filters einen Wert von etwa 30,000 Dalton beträgt.

2. Verfahren nach Anspruch 1, dadurch gekennzeichnet, daß ein Sack vorgesehen ist, der die Aszitesflüssigkeit aufnimmt, der eine Substanz zur Verhinderung der Blutgerinnung beigefügt wird, wobei eine Pumpe vorgesehen ist, die die Aszitesflüssigkeit aus dem Sack zu dem, im ersten Verfahrensschritt verwendeten Filter transportiert.

3. Verfahren nach wenigstens einem der vorangegangenen Ansprüche, dadurch gekennzeichnet, daß ein Sack auf der Ausgangsleitung des im ersten Verfahrensschritt verwendeten Filters angeordnet ist, aus dem die Aszitesflüssigkeit durch eine Pumpe abzogen wird, um dem im zweiten Verfahrensschritt verwendeten Filter zugeführt zu werden.

4. Verfahren nach wenigstens einem der vorangegangenen Ansprüche, dadurch gekennzeichnet, daß die gereinigte und konzentrierte Aszitesflüssigkeit verwendet wird, nachdem sie den im zweiten Verfahrensschritt verwendeten Filter durchsetzt hat.

5. Verfahren nach wenigstens einem der vorangegangenen Ansprüche, dadurch gekennzeichnet, daß die Aszitesflüssigkeit von dem im zweiten Verfahrensschritt verwendeten Filter wieder dem Sack zugeführt wird, der zwischen die im ersten bzw. im zweiten Verfahrensschritt verwendeten Filter eingefügt ist, und dann aus diesem Sack als Produkt abgezogen wird.

## Revendications

1. Procédé pour le traitement extracorporel du fluide ascitique, comportant une première étape dans laquelle le fluide traverse un filtre qui est capable d'éliminer du filtrat les particules, représentant des substances nocives, ayant un poids moléculaire supérieur à celui des substances qu'il est souhaité d'exploiter, et une seconde étape dans laquelle le filtrat de la première étape est concentré dans un filtre approprié avec perte d'eau, caractérisé en ce que la capacité de discrimination des poids moléculaires, ou exclusion, du filtre utilisé dans la première étape présente une valeur d'approximativement 200 000 daltons, tandis que la capacité de discrimination du filtre utilisé dans la seconde étape présente une valeur d'approximativement 30 000 daltons.

2. Procédé selon la revendication 1, caractérisé par la présence d'un sac qui reçoit le fluide ascitique dans lequel une substance anticoagulante est ajoutée, une pompe étant prévue qui est adaptée pour le transfert du fluide ascitique dudit sac au filtre utilisé dans la première étape.

3. Procédé selon une ou plusieurs des revendications précédentes, caractérisé par la présence d'un sac sur la ligne de sortie du filtre utilisé dans la première étape, à partir duquel le fluide ascitique est attiré par une pompe pour être envoyé au filtre utilisé dans la seconde étape.

4. Procédé selon une ou plusieurs des revendications précédentes, caractérisé en ce que le fluide ascitique purifié concentré est utilisé après avoir traversé le filtre employé dans la seconde étape.

5. Procédé selon une ou plusieurs des revendications précédentes, caractérisé en ce que le fluide ascitique est recirculé à partir du filtre employé dans la seconde étape vers le sac disposé entre les filtres employés respectivement dans les première et seconde étapes, puis est soutiré dudit sac en tant que produit.